# EUROPEAN PATENT APPLICATION

(11) **EP 1 894 939 A1**
(43) Date of publication of application: **05.03.2008**
(21) Application number: 06018384.5
(22) Date of filing: 01.09.2006
(51) Int. Cl.: C07K 1/22, C07K 14/00, C12N 15/00, C12N 5/00, G01N 33/58, G01N 33/53, C12N 15/62

(54) **Novel supertag and its use**

(71) Applicant: GSF-Forschungszentrum für Umwelt und Gesundheit GmbH, 85764 Neuherberg (DE)
(72) Inventor: Meisterernst, Michael, 82131 Stockdorf (DE); Kessler, Alexandra, 80805 München (DE)
(74) Representative: Bösl, Raphael Konrad

(57) **Abstract**

The present invention relates to an affinity marker comprising a first and a second tag, a protein comprising the affinity marker, a nucleic acid coding for the affinity marker or the protein, a vector comprising the nucleic acid, a cell comprising the nucleic acid or the vector, a method of determining or localizing a nucleic acid sequence or a method of purifying a protein with the affinity marker, the use of the affinity marker for the purification of a protein and/or for determining or localizing a protein or a nucleic acid sequence and a kit comprising the nucleic acid and/or the vector and optionally (a) suitable antibody/ies.

SEQUENCE LISTING

<110>  GSF-Forschungszentrum für Umwelt und Gesundheit GmbH

<120>  Novel Supermarker and its Use

<130>  G64456EP

<160>  4

<170>  PatentIn version 3.3

<210>  1
<211>  21
<212>  PRT
<213>  Artificial sequence

<220>
<223>  NC2alpha domain

<400>  1

Met Ser Pro Pro Thr Pro Phe Leu Pro Phe Ala Ser Thr Leu Pro Leu
1               5                   10                  15

Pro Pro Ala Pro Pro
            20

<210>  2
<211>  33
<212>  PRT
<213>  Artificial sequence

<220>
<223>  muted GCN2 domain

<400>  2

Arg Met Lys Gln Leu Glu Pro Lys Val Glu Glu Leu Leu Pro Lys Asn
1               5                   10                  15

Tyr His Leu Glu Asn Glu Val Ala Arg Leu Lys Lys Leu Val Gly Glu
            20                  25                  30

Arg

<210>  3
<211>  1117
<212>  DNA
<213>  Artificial sequence

<220>
<223>  Nucleic acid encoding MBP tag

<400>  3
agcttgcggc catgaaaact gaagaaggta aactggtaat ctggattaac ggcgataaag      60

gctataacgg tctcgctgaa gtcggtaaga aattcgagaa agataccgga attaaagtca     120

ccgttgagca tccggataaa ctggaagaga aattcccaca ggttgcggca actggcgatg     180

gccctgacat tatcttctgg gcacacgacc gctttggtgg ctacgctcaa tctggcctgt     240

tggctgaaat caccccggac aaagcgttcc aggacaagct gtatccgttt acctgggatg     300

ccgtacgtta caacggcaag ctgattgctt acccgatcgc tgttgaagcg ttatcgctga     360

tttataacaa agatctgctg ccgaacccgc caaaaacctg ggaagagatc ccggcgctgg     420

ataaagaact gaaagcgaaa ggtaagagcg cgctgatgtt caacctgcaa gaaccgtact     480

tcacctggcc gctgattgct gctgacgggg gttatgcgtt caagtatgaa aacggcaagt     540

acgacattaa agacgtgggc gtggataacg ctggcgcgaa agcgggtctg accttcctgg     600

ttgacctgat taaaaacaaa cacatgaatg cagacaccga ttactccatc gcagaagctg     660

cctttaataa aggcgaaaca gcgatgacca tcaacggccc gtgggcatgg tccaacatcg     720

acaccagcaa agtgaattat ggtgtaacgg tactgccgac cttcaagggt caaccatcca     780

aaccgttcgt tggcgtgctg agcgcaggta ttaacgccgc cagtccgaac aaagagctgg     840

caaaagagtt cctcgaaaac tatctgctga ctgatgaagg tctggaagcg gttaataaag     900

acaaaccgct gggtgccgta gcgctgaagt cttacgagga agagttggcg aaagatccac     960

gtattgccgc cactatggaa aacgcccaga aaggtgaaat catgccgaac atcccgcaga    1020

tgtccgcttt ctggtatgcc gtgcgtactg cggtgatcaa cgccgccagc ggtcgtcaga    1080

ctgtcgatga agccctgaaa gacgcgcaga ctggatc                             1117

<210>  4
<211>  57
<212>  PRT
<213>  Artificial sequence

<220>
<223>  Supertag NC2alpha domain- GSG-GCN2 domain

<400>  4

Met Ser Pro Pro Thr Pro Phe Leu Pro Phe Ala Ser Thr Leu Pro Leu
1               5                   10                  15

Pro Pro Ala Pro Pro Gly Ser Gly Arg Met Lys Gln Leu Glu Pro Lys
            20                  25                  30

Val Glu Glu Leu Leu Pro Lys Asn Tyr His Leu Glu Asn Glu Val Ala
        35                  40                  45

Arg Leu Lys Lys Leu Val Gly Glu Arg
    50                  55

## Description

The present invention relates to an affinity marker comprising a first and a second tag, a protein comprising the affinity marker, a nucleic acid coding for the affinity marker or the protein, a vector comprising the nucleic acid, a cell comprising the nucleic acid or the vector, a method of determining or localizing a nucleic acid sequence or a method of purifying a protein with the affinity marker, the use of the affinity marker for the purification of a protein and/or for determining or localizing a protein or a nucleic acid sequence and a kit comprising the nucleic acid and/or the vector and optionally (a) suitable antibody/ies.

In many areas of industry and research, nowadays pure or even ultrapure products are required, since product quality is often determined by their purity. This applies in particular to proteins produced by biotechnological methods, as these are now finding increasing application in a number of industrial products and processes. They are used for example as medicinal products, for diagnostic or scientific purposes. Furthermore, they are used in many areas of everyday life, for example as diet supplements, as additives in the food industry, for example as baking aids or in cheese-making, but also for example in papermaking, in the hygiene area or in detergents. Ultrapure proteins are in addition required for analytical methods, e.g. for elucidation of structure.

There are already many known methods by which products can be separated from other constituents. As a rule these make use of the differences in physical and chemical properties of the constituents of a sample that is to be purified. Conventional methods of purification and isolation include for example extraction, precipitation, recrystallization, filtration, centrifugation, washing and drying. The separation techniques and principles of adsorption, chromatography or ion exchange are also used. Various column materials are available for chromatographic methods, and make it possible to adapt the purification process to the particular product that is to be purified, making use of the differences in migration rates of the individual constituents, based for example on charge or hydrophobicity.

Affinity chromatography, in which a product, as a rule a protein, is separated and thus purified on the basis of its affinity for a binding partner, is especially suitable. So-called tags have now been developed, which are for example attached to a protein that is to be purified. The tag possesses an affinity for a particular binding partner; this can be utilized in affinity chromatography. Such tags are in principle of universal application and can be attached to various molecules. In this way it is possible to purify different molecules, especially proteins, with the same method of purification. Thus, ideally, the method does not need to be adapted specifically to the particular product.

Especially for analyzing and purifying proteins, the technique of affinity labeling, i.e. the attachment of a marker or tag to a protein by techniques of molecular biology, is now a frequently used method. In this technique, the primary sequence of any protein is expanded by just a few amino acids by means of recombinant techniques. The presence of a specific binding molecule with high affinity, e.g. an antibody, with a known recognition sequence, is decisive.

A number of (affinity) tags or (affinity) markers are known at present. These are usually divided into 3 classes according to their size: small tags have a maximum of 12 amino acids, medium-sized ones have a maximum of 60 and large ones have more than 60. The small tags include the Arg-tag, the His-tag, the Strep-tag, the Flag-tag, the T7-tag, the V5-peptide-tag and the c-Myc-tag, the medium-sized ones include the S-tag, the HAT-tag, the calmodulin-binding peptide, the chitin-binding peptide and some cellulose-binding domains. The latter can contain up to 189 amino acids and are then regarded, like the GST-and MBP-tag, as large affinity tags.

In order to produce especially pure proteins, so-called double tags or tandem tags were developed. In this case the proteins are purified in two separate chromatography steps, in each case utilizing the affinity of a first and then of a second tag. Examples of such double or tandem tags are the GST-His-tag (glutathione-S-transferase fused to a polyhistidine-tag), the 6xHis-Strep-tag (6 histidine residues fused to a Strep-tag (see below)), the 6xHis-tag100-tag (6 histidine residues fused to a 12-amino-acid protein of mammalian MAP-kinase 2), 8xHis-HA-tag (8 histidine residues fused to a haemagglutinin-epitope-tag), His-MBP (His-tag fused to a maltose-binding protein, FLAG-HA-tag (FLAG-tag fused to a hemagglutinin-epitope-tag), and the FLAG-Strep-tag. Most of these tag have been specifically developed for the purification of proteins produced by prokaryotic cells.

Often, however, it is necessary or desirable to purify proteins that were expressed by eukaryotic cells, e.g. if the protein is modified posttranslationally or proteins that are additionally present in the cell, and which bind to the target protein, are to be purified at the same time. The complexity of eukaryotic proteomes makes the purification of proteins that are expressed by eukaryotes challenging, and as a rule means that an individual purification protocol must be established for each protein. As a rule, therefore, the aforementioned double tags cannot be applied directly in eukaryotic systems.

Additionally, affinity tags are also used in the elucidation of nucleic acid sequences and their interaction with proteins. A recently developed technique includes Chromatin Immunoprecipitation (ChIP), a procedure used to determine whether a given protein binds to a specific DNA sequence in a eukaryotic cell or to localize a DNA sequence in a eukaryotic cell.

Therefore, double tags have already been developed for purification of proteins from eukaryotic cells as well. These encompass e.g. FLAG Strep tandem tags. In an experimental setup, the properties of various tags, e.g. HIS, CBP, CYD (covalent yet dissociable NorpD peptide), Strep II, FLAG, HPC (heavy chain of protein C), GST and MBP were compared with respect to their purification properties in various systems (Lichty et al., 2005, Protein Expr Purif 41: 98-105). The authors recommend, taking into account their results, a combination of 6xHis- and Strep II-tag for double purification.

However, the above methods often require a very high extent of purification capability demanding tags and antibodies binding to each other with high affinity.

The problem to be solved by the present invention was accordingly to provide an improved affinity marker, which is suitable for the purification of proteins, especially from eukaryotic cells, and/or for use in immunoprecipitation and ChIP techniques. In particular, a problem to be solved by the present invention was to provide an affinity marker for the purification of proteins from (eukaryotic) cells, offering maximum possible yields with preferably highest possible purity of the purified protein. In a preferred embodiment of the invention the affinity marker is intended for use in immunoprecipitation and chromatin immunoprecipitation techniques providing new potentials for improving detection and/or localization of proteins and/or nucleic acids. Further objects of the present invention relate to an affinity marker providing a standard or recovery marker, especially in immunoprecipitation and ChIP techniques.

The object of the present invention was solved by a new affinity marker comprising a first tag comprising a first epitope for an IgG antibody, wherein the first epitope is derived from a cellular protein, and a second tag comprising a second for an artificial antibody.

Accordingly, a first subject of the invention relates to an affinity marker comprising
- a first tag comprising a first epitope for an IgG antibody, wherein the first epitope is derived from a cellular protein; and
- a second tag comprising a second epitope for an artificial antibody.

Surprisingly, it was found that an affinity marker comprising a combination of an epitope for an IgG antibody, wherein the first epitope is derived from a cellular protein, and an artificial antibody is superior to the existing double tag or tandem affinity markers.

The present invention provides a new concept of tandem affinity markers. The affinity marker involves two different separation steps, since two completely different types of epitopes for two different classes of antibodies (IgG and an artificial antibody) are used, which leads to a surprisingly high degree of separation and/or purification. In addition to very high affinities of the respective antibodies for the prevailing epitope, which already provides a quite high degree of separation/purification, the change of purification partners for each of the epitopes from IgG to an artificial antibody or *vice versa* provides for an additional purification effect. The diversity of the natures of the antibodies leads to different kinds of impurities in each of the purification steps, which the other purification step compensates for, which in return results in a very high purity at comparably high yields.

As detailed above, one of the epitopes of the affinity marker is an epitope for an IgG antibody. An IgG antibody is a monomeric immunoglobulin, built of two heavy chains and two light chains. Each molecule has two antigen binding sites. In the present invention the affinity marker comprises an epitope for an IgG antibody and is derived from a cellular protein.

"Derived from a cellular protein" in the present context relates to a protein which is identical to or related to a cellular protein, wherein "related to a cellular protein" also refers to a portion of a cellular protein sufficient to be specifically recognized by an IgG antibody. Preferably the portion of the cellular protein encompasses at least 10 consecutive amino acids of that cellular protein, preferably at least 12, more preferably at least 15, still more preferably at least 17 and most preferably at least 20 or 21 consecutive amino acids of the cellular protein. Also encompassed by the term "related to a cellular protein" is a full length cellular protein or a portion of a cellular protein as defined above in which at most about 10 %, more preferably at most about 5 %, still more preferably at most about 1 % of the amino acids have been substituted for another amino acids, more preferably wherein the substitution is a conservative amino acid substitution.

Conservative amino acid substitutions are those that take place within a family of amino acids that are related in their side chains and chemical properties. Examples of such families are amino acids with basic side chains, with acidic side chains, with non-polar aliphatic side chains, with non-polar aromatic side chains, with uncharged polar side chains, with small side chains, with large side chains etc.. Examples of conservative amino acid substitutions include, but are not limited to, those listed below:

| Original Residue | Conservative Substitutions |
|---|---|
| Ala | Ser |
| Arg | Lys |
| Asn | Gln; His |
| Asp | Glu |
| Cys | Ser |
| Gln | Asn |
| Glu | Asp |
| His | Asn; Gln |
| Ile | Leu, Val |
| Leu | Ile; Val |
| Lys | Arg; Gln; Asn |
| Met | Leu; Ile |
| Phe | Met; Leu; Tyr |
| Ser | Thr |
| Thr | Ser |
| Trp | Tyr |
| Tyr | Trp; Phe |
| Val | Ile; Leu |

By "cellular protein" is meant a protein which is produced by a naturally occurring cell. The protein may be present in the cell until e.g. being degraded or otherwise chemically altered or may be released from that cell after being produced e.g. in order to act on or in a different cell.

Examples of such proteins include enzymes, e.g. a oxidoreductase, a transferase, a hydrolyse, a lyase, a isomerase, a ligase, a kinase or a lipase, a dehydrogenase, a reductase, an oxidase, a synthase, a dismutase, a phosphorylase, a transaminase, an esterase, a peptidase, a decarboxylase, a dehydratase, a racemase, an isomerase, an epimerase or a mutase; proteins involved in the signal transduction, such as various kinases and phosphatases, e.g. PKA or PKC, adenylyl cyclase, a tyrosine kinase, transcription factors such as TFIIA, TFIIB, TFIID, TFIIE, TFIIF, TFIIH, E2F1, E2F2, E2F3a, E2F3b, E2F4, E2F5, E2F6, E2F7 or E2F8, factors regulating expression of a gene or PLC; proteins involved in regulation of the cell cycle; proteins for transporting a compound; structural cellular proteins such as actin or tubulin or membrane proteins such as receptors or channels.

In a preferred embodiment the affinity marker of the invention is characterized in that the first epitope is derived from a cellular protein of a eukaryotic cell, preferably a vertebrate cell, more preferably a mammalian cell, still more preferably a human cell, wherein the above definition of "derived from" is to be applied analogously.

In an even more preferred embodiment the affinity marker of claim of the invention is not present in prokaryotic cells.

Yet in another more preferred embodiment of the invention the cellular protein is derived from a protein regulating expression, preferably transcription of a gene, more preferably a protein capable of binding to a sequence directing the transcription of a gene such as a promoter or enhancer, e.g. a transcription factor. Examples of transcription factors are mentioned above.

In an even more preferred embodiment the affinity marker of the invention is characterized in that the cellular protein is a protein capable of binding DNA, optionally a complex of DNA and one or more proteins, more preferably the TATA box binding protein (TBP) - TATA Element Complex.

In a still more preferred embodiment the affinity marker of the invention is characterized in that the cellular protein is derived from negative co-factor 2 (NC2), preferably NC2α. NC2α is also referred to as Dr1-associated corepressor. The human amino acid sequence of this factor is available to the public, e.g. at the NCBI (Accession number Q14919) and has also been published (Goppelt et al., 1996, EMBO J. 15: 3105-3116). The IgG antibody is preferably directed against an epitope which is located in a portion of NC2α, more preferably of human NC2α, especially in a prolin-rich portion of (human) NC2α which is located close to the N-terminal region.

In a highly preferred embodiment of the invention the first tag comprises or consists of the sequence:
Met Ser Pro Pro Thr Pro Phe Leu Pro Phe Ala Ser Thr Leu Pro Leu Pro Pro Ala Pro Pro (SEQ ID NO:1)
(i.e. methionin plus amino acids 171 to 190 of human NC2α)

However, the tag may differ from the sequence of SEQ ID NO:1 by at most 5, preferably at most 4, more preferably at most 3, still more preferably at most 2, most preferably at most 1 amino acid addition, deletion and/or substitution. In case of substitution one or more conservative amino acid substitution(s) are preferred, wherein the conservative amino acid substitution is as defined above. The one or more amino acid addition(s), deletion(s) and/or substitution(s) may be C-terminally, N-terminally or internally as well as combinations thereof. If more than one amino acid is altered by addition(s), deletion(s), substitution(s) or a combination thereof, the two or more mutations are preferably separated by at least 3, 4 or 5, more preferably at least 10 or 15 unchanged amino acids. Additionally, one or more C-terminal or N-terminal mutation(s)/alteration(s) is/are preferred compared as to one or more internal mutation(s).

The affinity marker is further characterized by the presence of a second tag comprising a second epitope for an artificial antibody. Each of the tags comprises or consists of the respective epitope; the tags may encompass additional elements, in general it consists of the epitope and optionally one or more additional amino acids.

An artificial antibody in the context of the present invention is an antibody whose structure is different from naturally occurring antibodies such as IgA, IgD, IgE IgG or IgM, the structure of which is well known and characterized and is based on a Y shape. Artificial antibodies are in general characterized by a high affinity binding to their epitopes as compared to naturally occurring or traditional antibodies, since their structure has been adapted and improved for this purpose (increasing affinity).

The artificial antibody may be a single chain antibody, which is smaller in size than the naturally occurring antibodies. The non-natural antibody format of single chain antibodies (SCAs or scFvs) combines essentially only the antigen-binding regions of antibodies on a single stably-folded polypeptide chain. As such, single chain antibodies are of considerably smaller size than classical immunoglobulins but retain the antigen-specific binding properties of antibodies. Consequently, SCAs provide attributes which traditional antibodies cannot deliver. A major obstacle of methods employing traditional antibodies is the non-specific background generated by both the binding and non-binding regions of the intact antibodies. However, protein fragments consisting of the minimal binding subunit of antibodies, i.e. single chain antibodies, have excellent binding specificity and affinity for their epitopes. In contrast to traditional antibodies, single chain antibodies lack the non-binding regions, can be selected in the company of competing antigens, and therefore have potential for higher specificity/sensitivity in separation and purification methods. The production of a scFv directed against GCN4 is exemplified in Zahnd et al, 2004, J Biol Chem. 279: 18870-18877.

Single chain antibodies and their production are disclosed in a series of patents and patent applications including e.g. EP 0281604, US 4,946,778, US 5,260,203, US 5,455,030, US 5,518,889, US 5,534,621, US 4,704,692, US 5,658,763, US 5,631,158, US 5,733,782, EP 0318554, EP 0623679 and US 6,103,889.

Another example of an artificial antibody is a (designed) ankyrin repeat protein. These proteins are disclosed in WO 02/20565. In general the ankyrin repeat protein encompasses repeat domains between capping repeats, which are special terminal repeats of ankyrin repeats. Optional elements are linkers to join further domains such as a further series of repeat domains or a non-repeat domain. Examples of typical ankyrin repeat sequence motifs include without limitation:
- DxxGxTPLHLAaxx±±±±±±±±±±GpxpaVpxLLpxGA±±±±±DVNAx, wherein "x" denotes any amino acid, "±"denotes any amino acid or a deletion, "a" denotes an amino acid with an apolar side chain, and "p" denotes a residue with a polar side chain,
- DxxGxTPLHLAxxxGxxxVVxLLLxxGADVNAx, wherein "x" denotes any amino acid
- DxxGxTPLHLAxxxGxxxlVxVLLxxGADVNAx, wherein "x" denotes any amino acid and
- D11G1TPLHLAA11GHLEIVEVLLK2GADVNA1, wherein 1 represents an amino acid residue selected from the group: A, D, E, F, H, I, K, L, M, N, Q, R, S, T, V,W and Y; wherein 2 represents an amino acid residue selected from the group: H, N and Y.

The production and selection of a high affinity designed ankyrin repeat protein is disclosed in Binz et al., 2004, Nature Biotechnology 22: 575-582.

In a preferred embodiment of the invention the second epitope is derived from a protein which is not present in a human cell, preferably a mammalian cell, more preferably a vertebrate cell, still more preferably an animal cell, wherein "derived from" has the meaning as defined above.

In another preferred embodiment of the invention the second epitope is derived from a protein of a fungal cell such as a cell of *Rhizomucor, Saccharomyces, Schizoaccharomyces* or *Aspergillus,* preferably a yeast cell, more preferably from *Saccharomyces cerevisiae.*

In a preferred embodiment of the invention the protein is or is derived from a transcription factor, preferably a transcription factor responsible for the activation of one or more genes required for amino acid or for purine biosynthesis.

In a even more preferred embodiment of the invention the second epitope is derived from GCN4, particularly *Saccharomyces cerevisiae* GCN4, the sequence of which is public available at NCBI (Accession number AAL09032) and has been published by Czerwinski et al., 2002, Transfusion 42: 257-264.

The epitope may differ from the above sequence as published (see Czerwinski et al., *supra*) by at most 5, preferably at most 4, more preferably at most 3, still more preferably at most 2, most preferably at most 1 amino acid addition, deletion and/or substitution. In case of substitution one or more conservative amino acid substitution(s) are preferred, wherein the conservative amino acid substitution is as defined above. The one or more amino acid addition(s), deletion(s) and/or substitution(s) may be C-terminally, N-terminally or internally as well as combinations thereof. If more than one amino acid is altered by addition(s), deletion(s), substitution(s) or a combination thereof, the two or more mutations are preferably separated by at least 3, 4 or 5, more preferably at least 10 or 15 unchanged amino acids. Additionally, one or more C-terminal or N-terminal mutation(s)/alteration(s) is/are preferred compared to one or more internal mutation(s).

An especially preferred second tag comprises or consists of the sequence Arg Met Lys Gln Leu Glu Pro Lys Val Glu Glu Leu Leu Pro Lys Asn Tyr His Leu Glu Asn Glu Val Ala Arg Leu Lys Lys Leu Val Gly Glu Arg (SEQ ID NO:2), which differs from the sequence as published (see Czerwinski et al., *supra*) by deletion of the 2 C-terminal amino acids (T and S) and substitution of amino acid 7 and 14, wherein D and S, respectively, are replaced with P.

As detailed above, the second epitope may be an epitope for an ankyrin repeat protein as defined above. An especially preferred second tag encompassing an epitope for an ankyrin repeat protein comprises or consists of the sequence of maltose-binding protein (MBP). Particularly, the nucleic acid sequence encoding the tag may be

AGCTTGCGGCCATGAAAACTGAAGAAGGTAAACTGGTAATCTGGATTAA
CGGCGATAAAGGCTATAACGGTCTCGCTGAAGTCGGTAAGAAATTCGAG
AAAGATACCGGAATTAAAGTCACCGTTGAGCATCCGGATAAACTGGAAG
AGAAATTCCCACAGGTTGCGGCAACTGGCGATGGCCCTGACATTATCTTC
TGGGCACACGACCGCTTTGGTGGCTACGCTCAATCTGGCCTGTTGGCTGA
AATCACCCCGGACAAAGCGTTCCAGGACAAGCTGTATCCGTTTACCTGGG
ATGCCGTACGTTACAACGGCAAGCTGATTGCTTACCCGATCGCTGTTGAA
GCGTTATCGCTGATTTATAACAAAGATCTGCTGCCGAACCCGCCAAAAAC

CTGGGAAGAGATCCCGGCGCTGGATAAAGAACTGAAAGCGAAAGGTAA
GAGCGCGCTGATGTTCAACCTGCAAGAACCGTACTTCACCTGGCCGCTGA
TTGCTGCTGACGGGGGTTATGCGTTCAAGTATGAAAACGGCAAGTACGA
CATTAAAGACGTGGGCGTGGATAACGCTGGCGCGAAAGCGGGTCTGACC
TTCCTGGTTGACCTGATTAAAAACAAACACATGAATGCAGACACCGATTA
CTCCATCGCAGAAGCTGCCTTTAATAAAGGCGAAACAGCGATGACCATC
AACGGCCCGTGGGCATGGTCCAACATCGACACCAGCAAAGTGAATTATG
GTGTAACGGTACTGCCGACCTTCAAGGGTCAACCATCCAAACCGTTCGTT
GGCGTGCTGAGCGCAGGTATTAACGCCGCCAGTCCGAACAAAGAGCTGG
CAAAAGAGTTCCTCGAAAACTATCTGCTGACTGATGAAGGTCTGGAAGC
GGTTAATAAAGACAAACCGCTGGGTGCCGTAGCGCTGAAGTCTTACGAG
GAAGAGTTGGCGAAAGATCCACGTATTGCCGCCACTATGGAAAACGCCC
AGAAAGGTGAAATCATGCCGAACATCCCGCAGATGTCCGCTTTCTGGTAT
GCCGTGCGTACTGCGGTGATCAACGCCGCCAGCGGTCGTCAGACTGTCGA
TGAAGCCCTGAAAGACGCGCAGACTGGATC
(SEQ ID NO:3)

However, in another embodiment the second tag may differ from the sequence of SEQ ID NO:2 or the sequence encoded by SEQ ID NO: 3 by at most 10, preferably at most 5, more preferably at most 3, still more preferably at most 2, most preferably at most 1 amino acid addition, deletion and/or substitution. In case of substitution one or more conservative amino acid substitution(s) are preferred, wherein the conservative amino acid substitution is as defined above. Alternatively substitutions replacing any amino acid with prolin are also preferred. The one or more amino acid addition(s), deletion(s) and/or substitution(s) may be C-terminally, N-terminally or internally as well as combinations thereof. If more than one amino acid is altered by addition(s), deletion(s), substitution(s) or a combination thereof, the two or more mutations are preferably separated by at least 3, 4 or 5, more preferably at least 6, 7, 8, 9 or 10 unchanged amino acids. Additionally, one or more C-terminal or N-terminal mutation(s)/alteration(s) is/are preferred as compared to one or more internal mutation(s).

In an even more preferred embodiment of the invention, the MBP epitope may be combined with an NC2 epitope, more preferably an NC2 epitope as defined above. A highly preferred affinity marker may be composed as follows:
Optionally linker/cloning site - Tag with MBP epitope (second epitope) - linker/cloning site - Tag with IgG epitope (first epitope) - optionally linker/cloning site,
   or
Optionally linker/cloning site - Tag with epitope for artificial antibody (second epitope) - linker/cloning site - Tag with NC2 epitope (first epitope) - optionally linker/cloning site,
   particularly
Optionally linker/cloning site - Tag with MBP epitope (second epitope) - linker/cloning site - Tag with NC2 epitope (first epitope) - optionally linker/cloning site
   especially
Tag with MBP epitope (second epitope) - linker/cloning site - Tag with NC2 epitope (first epitope)

Within the affinity marker both tags may be directly joined to each other or may be joined by a linker. The linker can be any suitable linker, especially a linker of amino acid residues. Preferred linkers are short peptide linkers consisting of 1, 2, 3, 4, 5, 6, 7, 8, 9 or 10 amino acids, preferably 1, 2, 3, 4 or 5 amino acids, more preferably 1, 2 or 3 amino acids. Preferably each of these amino acids is Gly or Ser or combinations thereof. A particular preferred linker comprises or consists of the amino acid sequence Gly Ser Gly.

In a preferred embodiment of the invention the affinity marker comprises or consists of the tags of SEQ ID NO: and SEQ ID NO:2, more preferably joined by a linker, especially Gly Ser Gly. The affinity marker may comprise or consist of the sequence Met Ser Pro Pro Thr Pro Phe Leu Pro Phe Ala Ser Thr Leu Pro Leu Pro Pro Ala Pro Pro Gly Ser Gly Arg Met Lys Gln Leu Glu Pro Lys Val Glu Glu Leu Leu Pro Lys Asn Tyr His Leu Glu Asn Glu Val Ala Arg Leu Lys Lys Leu Val Gly Glu Arg (SEQ ID NO:4).

In one embodiment of the invention it may be necessary or desirable to cleave off at least one of the two tags of a tagged protein. In this case a cleavage site, for example a cleavage site for an enzyme, may be present. The cleavage site could for example be a protease cleavage site. Examples of proteases are chymotrypsin, trypsin, elastase, and plasmin; the corresponding cleavage sites are known to a person skilled in the art. Since the molecule tagged is a protein, specific proteases, especially proteases from viruses that normally attack plants, are preferred. Examples of suitable specific proteases are thrombin, factor Xa, Igase, TEV-protease from the "Tobacco Etch Virus", the protease PreScission (Human Rhinovirus 3C Protease), enterokinase or Kex2. TEV-protease and PreScission are especially preferred.

The affinity marker according to the present invention can be produced for example by chemical synthesis in a known manner, e.g. by solid phase synthesis of linear peptide building blocks (SPPS) according to Merrifield using suitable protecting groups such as Fmoc. Individual components can also be joined together via peptide bonds. For this purpose it is possible to use e.g. amino acid coupling by activation of the carboxylic acid using for example 1-hydroxy-1H-benzotriazole (HOBt) and carbodiimide (e.g. EDC).

Another subject of the invention relates to a protein comprising the affinity marker of the invention, wherein the affinity marker may be any of the affinity markers as defined above. A protein according to the present invention is a polymer composed of amino acids, with the individual amino acids joined together by peptide bonds to form chains. The length of the amino acid chains can be more than 10000 amino acids, and is preferably more than 50 or more than 100 amino acids. The affinity marker that is bound to the protein to be purified or used in the methods of the invention is produced in a suitable manner by the techniques of genetic engineering using the corresponding nucleic acid and/or a suitable vector. These techniques are well known to a person skilled in the art and are described in more detail below. The protein contains both tags, optionally a cleavage site, and the protein to be purified (protein sample).

The arrangement of the individual components in the protein with affinity marker, the underlying nucleic acid or the underlying vector can be as follows, for example:
First tag-protein-Second tag,
Second tag-protein- First tag,
First tag-Second tag -protein,
Second tag-First tag -protein,
Protein-First tag-Second tag or
Protein-Second tag-First tag.

A further subject of the present invention is a nucleic acid that codes for a protein containing the affinity marker according to the invention. The nucleic acid may be, for example, an RNA or DNA. These can be used for example for production of the protein, labelled with the affinity marker that is to be purified. For this, the nucleic acid can be inserted in a cell, especially a eukaryotic cell, and expressed in the cell. Methods for the insertion and expression of nucleic acids in cells are generally familiar to a person skilled in the art. A nucleic acid comprising the any of the sequences of SEQ ID NO: 1 to 4 is especially preferred.

Vectors are usually used for the production of proteins, especially of recombinant proteins. Accordingly, yet another subject of the present invention is a vector containing a nucleic acid according to the invention. These vectors are then inserted in a target cell and the target cell is cultivated. With selection of a suitable vector, the target cell produces the desired protein. A great many vectors are known in the prior art. As a rule the vector is selected in relation to the target cell, in order to achieve expression that is as efficient as possible. In addition to the nucleic acid for the affinity marker optionally in combination with that for the protein of interest, the vector contains e.g. a suitable promoter, enhancer, selection marker and/or a suitable signal sequence, which for example causes the protein to be secreted into the medium, and optionally suitable cleavage sites for e.g. restriction enzymes. The constituents of vectors are familiar to a person skilled in the art, who will be able to select them in relation to the particular target cell. The restriction enzyme cleavage sites make it possible for nucleic acids that code for various proteins of interest to be incorporated in the vector and removed simply and quickly. Depending on what side the affinity marker is to be bound to the protein subsequently, they can be located 5' or 3' from the nucleic acid that codes for the affinity marker, or between the nucleic acid segments encoded by the first and second tag.

The protein contains the components first tag and second tag plus the protein to be purified or intended for the methods of the invention, each of which are as defined above and can also be arranged thus. Additionally, there may be for example a linker between the two domains, one or more cleavage sites or a signal sequence. The individual components are arranged in the protein as explained above. A person skilled in the art knows how to derive a nucleic acid sequence from a protein sequence, taking into account the genetic code. He also knows how to produce such a nucleic acid sequence using standard techniques of molecular biology. This can be accomplished for example by the use and combination of existing sequences using restriction enzymes. The nucleic acid suitably also contains further elements e.g. a promoter, enhancer, a transcription start and stop signal and a translation start signal.

The nucleic acid thus produced will then, optionally by means of a vector, be inserted and expressed in a target cell. Accordingly, a further subject of the present invention is a cell containing a nucleic acid according to the invention or a vector according to the invention. The target cell can be any suitable cell, especially a eukaryotic cell, for example a fungal, plant or animal cell. Cell lines of these cells are also included. Preferably it is a mammalian cell, especially a human cell or cell line. Examples of such cells are HEK 293 cells, CHO cells, HeLa cells, CaCo cells, Raji cells or NIH 3T3 cells. Suitable vectors can be used for transfection of the cells. Examples of vectors are pBR322, the pUC series, pBluescript, pTZ, pSP, pREP4 and pGEM. The components of the nucleic acid or of the vector are selected in such a way that the nucleic acid is expressed and the target protein (affinity marker and protein of interest) is produced by the target cell. The cells are cultivated until a sufficient amount of target protein has been produced. Expression and cultivation may e.g. be performed as described in Examples 1 or 2.

Then the protein can be isolated. If a sufficient amount of the target protein has been secreted into the medium, work can continue with this. Otherwise it may be necessary to disrupt the cells. This can be effected for example by lysis of the cells e.g. by means of ultrasound or hypotonic medium. To remove insoluble components, the sample obtained can for example be centrifuged, especially at 10000 x g to 15000 x g, and the supernatant obtained can be used further for the method of purification according to the invention (see below).

Another subject of the invention relates to the use of the affinity marker of the invention for immunoprecipitation or chromatin immunoprecipitation.

Immunoprecipitation (IP) is one of the most widely used immunochemical techniques. Immunoprecipitation followed by e.g. SDS-PAGE and immunoblotting, is routinely used e.g. to study protein/protein interactions, to determine specific enzymatic activity or to determine the presence and quantity of proteins. The IP technique also enables the detection of rare proteins which otherwise would be difficult to detect since they can be concentrated up to 10,000-fold by immunoprecipitation.

In the IP method, the protein from the cell or tissue homogenate is precipitated in an appropriate buffer by means of an immune complex which includes the binding of a primary antibody to its epitope and the binding of a Protein A-, G-, or L-agarose conjugate or a secondary antibody-agarose conjugate. The choice of agarose conjugate depends on the species origin and isotype of the primary antibody. The methods described are comparable and the choice of method depends on the specific antigen-antibody system. In general, methods of performing immunoprecipitation are well known to the skilled practitioner and can be easily adapted when employing the affinity marker of the invention. However, immunoprecipitation may also be performed as described in Example 3.

Chromatin immunoprecipitation (ChIP) is a method to determine whether proteins including (but not limited to) transcription factors bind to a particular region on the endogenous chromatin or on the DNA of living cells or tissues, wherein this test is in general performed using living cells.

ChIP involves the immunoprecipitation of protein/DNA complexes that have been stabilized via cross-linking. Traditionally, transcription factor and gene promoter activity has been analyzed using reporter gene assays, EMSA, Western blot and DNA microarrays. Although these methods have led to significant advances in the scientific understanding of transcription, they cannot be used to demonstrate that a particular protein is bound to a specific, native DNA sequence in living cells. ChIP offers a versatile solution by combining the specificity of immunoprecipitation, the sensitivity of PCR and the screening power of array profiling, all in a single assay.

The principle underpinning this assay is that DNA-bound proteins (including transcription factors) in living cells can be cross-linked to the chromatin on which they are situated. This is usually accomplished by e.g. a gentle formaldehyde fixation. Following fixation, the cells are lysed, if living cells are used, and the DNA is broken into pieces such as pieces of e.g. 0.2-1 kb in length by e.g. sonication. Once the protein is immobilized on the chromatin and the chromatin is fragmented, whole protein-DNA complexes can be immunoprecipitated using an antibody specific for the protein in question. The DNA from the isolated protein/DNA fraction can then be purified. The identity of the DNA fragments isolated in complex with the protein of interest can then be determined by PCR using primers specific for the DNA regions that the protein in question is hypothesized to bind. Alternatively, when one wants to find where the protein binds across the whole genome, a DNA microarray can be used (ChIP on chip or ChIP-chip) allowing for the characterization of the cistrome. However, the ChIP requires high affinity antibodies having a specificity which are difficult and expensive to produce for each protein of interest. Therefore, affinity markers are conventionally used and linked to the protein of interest (in other words the protein of interest is labeled with an affinity marker) and used for purification or detection reason.

In general ChIP is performed on intact cells, which express the protein of interest linked to the affinity marker of the invention. First, cells may be fixed with formaldehyde, which cross-links and therefore preserves protein/DNA interactions. DNA is then sonicated into small uniform fragments and the DNA/protein complexes are immunoprecipitated using an antibody directed against the DNA-binding protein of interest with is tagged with the affinity marker of the invention. Following immunoprecipitation, cross-linking is reversed, proteins are removed e.g. by Proteinase K treatment and the DNA is cleaned up using the included DNA purification columns. The DNA is then screened to determine which genes were bound by the protein of interest. The versatility of ChIP means that screening can be done using generalized hybridization or a more targeted PCR-based approach.

For successful ChIP, it may be necessary to shear the chromatin to fragments, e.g. 200-1000 bp fragments. This has traditionally been performed by subjecting the isolated chromatin to different pulses of sonication (e.g. as detailed in the Examples). Alternatively a "Enzymatic Shearing Kit" may be used which shears DNA into fragments.

Thereafter, cells may be fixed (e.g for 10 minutes with 1% formaldehyde or as detaield in the Examples) and then chromatin may be sheared as described above (e.g as detailed in the Examples or using a commercially available "Enzymatic Shearing Kit"). The sheared and/or unsheared chromatin samples may be subjected to cross-link reversal, treated with Proteinase K, phenol/chloroform extracted and precipitated using the second epitope and the artificial antibody. Samples may be separated a second affinity purification using the first epitop and the correspomnding antibody. by electrophoresis e.g. through a 1% agarose gel. Positive and negative controls may be included into the assay. After separation by electrophoresis the obtained sequences may be analyses e.g. by PCR using suitable primers.

Accordingly, another subject of the present invention relates to a method of determining or localizing a nucleic acid sequence comprising
a) incubating a protein comprising the affinity marker of the invention with a nucleic acid under conditions allowing the formation of a protein nucleic acid complex;
b) optionally producing fragments of the nucleic acid of the complex of step a);
c) purifying the complex of step a) or b) by means of affinity purification using an antibody against the second epitope; and
d) determining and/or localizing the nucleic acid sequence bound to the protein,
wherein the first epitope of the affinity marker is used for pre-purification of the protein nucleic acid complex of step a) or b) prior to step c) and/or is used for standardization.

As detailed above, the first epitope is derived from a cellular protein or identical to a cellular protein. Accordingly, the ChIP may encompass two affinity purification steps, wherein the complex is first purified using the first epitope. The resulting purified solution encompasses the complex of tagged protein and DNA as well as the cellular protein produced by the cell due to its normal function. The amount of both may be determined before and after this first purification step. Thereafter, the complex may be purified using the second tag. Accordingly, the cellular protein is removed. The amount of complex may be determined again, wherein the difference of both amounts allows for standardization. In a similar manner the affinity marker may be used as recovery marker.

Each of the steps of the ChIP assay may independently be carried out e.g. as described in Example 4.

A further subject of the present invention is a method for the purification of a protein, especially expressed in a (eukaryotic) cell, preferably a mammalian cell, comprising the steps:
a) providing the protein according to the invention;
b) purifying the protein of step a) by means of affinity purification using an IgG antibody; and
c) purifying the purified protein from step b) by means of affinity purification using an artificial antibody.

Alternatively, the following method can also be used:
a) providing the protein according to the invention;
b) purifying the protein of step a) by means of affinity purification using an artificial antibody; and
c) purifying the purified protein from step b) by means of affinity purification using an IgG antibody.

The sample containing the protein labeled with the affinity marker can be obtained as described above, for example using genetic engineering by expression in a cell, especially a eukaryotic cell.

The sample, obtained as described above, is purified by tandem affinity purification using the first tag and the second tag and the respective antibody (IgG or artificial antibody). Affinity purification is a special form of adsorption purification, in which there are, on a carrier, groups (binding partners) with high affinity and therefore high binding strength to one of the two domains, so that these can be adsorbed preferentially and thus separated from other substances. Purification can be carried out either first via the first tag and then via the second tag, or *vice versa.* Purification takes place by specific binding to a suitable binding partner. The binding partner is preferably bound to a solid phase. The solid phase can be usual carrier materials, for example Sepharose, Superflow, Macroprep, POROS 20 or POROS 50. Separation is then carried out for example chromatographically, e.g. by gravity, HPLC or FPLC. Alternatively, the binding partner can also be bound to beads, especially magnetic beads. After adding the beads to the sample, binding takes place between the particular domain and the corresponding binding partner. The suspension can then be centrifuged for example, so that the labeled molecule sediments with the bead, and other components remain in the supernatant, from where they can be removed. Alternatively, the suspension is separated utilizing the magnetic properties of the beads. In one embodiment, the suspension is applied to a column, which is located in a magnetic field. As the magnetic beads and the molecule bound to them are retained in the magnetic field, other constituents of the sample can be washed out in several washing operations. The protein of interest can then for example be washed from the beads using a suitable elution buffer, or can be separated from the beads by enzymatic cleavage e.g. at the cleavage site between the two tags.

The first purification of the protein (step b)) takes place by a) binding of the first tag (or the second tag) to the respective binding partner (see above) in suitable conditions, b) optionally washing and c) detachment of the protein from the binding partner. The latter can either be effected by altering the conditions, so that the changed conditions no longer permit binding between affinity marker and binding partner (e.g. alteration of the pH value or the ionic strength), or by separating the molecule from the domain bound to the binding partner. Separation can be effected by cleavage of the bond between tag and binding partner, e.g. by chemical means or using specific enzymes, as was described in detail above. Alternatively, it is also possible to use specific competitors, which are added in excess.

This is followed by the second purification of the protein (step c)) by a) binding of the second tag (or the first tag), i.e. the tag that was not used in the first purification step, to the respective binding partner in suitable conditions, b) optionally washing and c) detachment of the tag from the binding partner, where these steps can be of the same form as those described for the first purification step.

The binding partner is the respective antibody as detailed above. The IgG antibody used can either be a polyclonal or preferably monoclonal antibody produced by methods known to a person skilled in the art or an antibody known from the prior art. In this case elution of the bound protein can be carried out for example with the competitors such as synthetic peptides.

Preferably, the artificial antibody used in the methods of the present invention is a single chain antibody or an ankyrin repeat protein as defined above. More preferably the single chain antibody is the scFv antibody 52SR4 (Zahnd et al., supra), which may be used if the second epitope or tag consist of or comprises GCN4, preferably GCN4(7P14P) (SEQ ID NO: 2).

The antibodies of the present invention are particularly preferred, if they are capable of binding to the epitope with very high affinity. Accordingly, the IgG antibody may bind the first epitope of the affinity marker of the invention with an affinity at most 100 x 10⁻⁹ mol/l, preferably at most 50 x 10⁻⁹ mol/l, more preferably 10 x 10⁻⁹ mol/l, still more preferably 5 x 10⁻⁹ mol/l, even more preferably 3 x 10⁻⁹ mol/l, most preferably 1 x 10⁻⁹ mol/l. Additionally, or alternatively, the artificial antibody may bind the second epitope of the affinity marker of the invention with an affinity at most 100 x 10⁻¹² mol/l, preferably at most 50 x 10⁻¹² mol/l, more preferably 10 x 10⁻¹² mol/l, still more preferably 5 x 10⁻¹² mol/l, even more preferably 3 x 10⁻¹² mol/l, most preferably 1 x 10⁻¹² mol/l.

General methods of determining the affinity of an antibody to its epitope are well known to the skilled person and may be adapted to the present epitopes and antibodies.

In one embodiment of the invention the labeled or tagged protein to be purified or used in the methods of the invention may have been or may be produced by a eukaryotic cell, preferably a vertebrate cell, more preferably a mammalian cell, still more preferably a human cell.

Additionally, at least one of the tags may be cleaved off after step b), c) and/or step d) of the methods of the present invention, as appropriate.
Furthermore, the IgG antibody and/or the artificial antibody used in the context of the present invention may be attached to a microarray or chip.

In a preferred embodiment of the invention the first epitope is used for standardization and/or as recovery marker as detailed above in the context of ChIP.

In one embodiment of the invention, the method of purification according to the invention can also be used in order to identify components (prey) which interact with a molecule labeled with the affinity marker (bait) and bind to it. Such bait/prey experiments are familiar to a person skilled in the art.

Yet another subject of the invention relates to the use of the affinity marker of the invention for the purification of a protein and/or for determining or localizing a protein or nucleic acid sequence, particularly from a cell, especially from a eukaryotic cell, preferably from a mammalian cell..

Still another subject of the present invention is a kit comprising
- the nucleic acid and/or the vector of the invention as defined above; and
- optionally an IgG antibody specific for the first epitope as defined above; and
- optionally an artificial antibody specific for the second epitope as defined above.

Additionally, the kit may encompass instructions for carrying out the e.g. the methods of the present invention.

The present invention is additionally described by the following figures and examples, which are not to be interpreted as limiting the scope of protection of the present invention.

### FIGURES

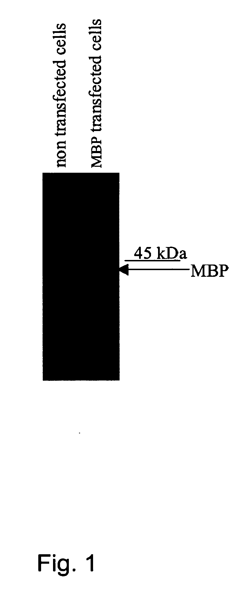

- **Fig. 1**: shows a Western blot analysis of MBP from Raji cells of EXAMPLE 2. 50 µg total protein prepared from MBP-transfected or non transfected Rajji cells was separated on an 12% SDS PAGE and transfered onto nitrocellulose. Membrane was probed with anti-MBP antisera (NEB).

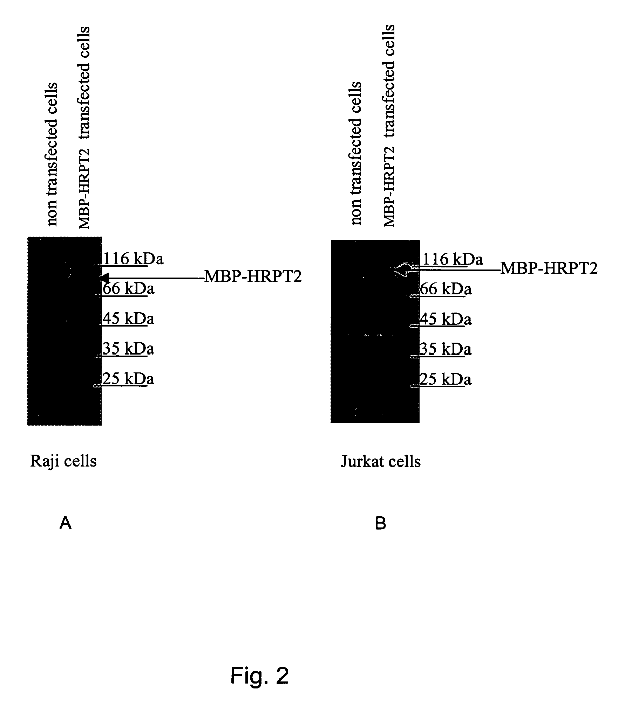

- **Fig. 2**: shows a Western blot analysis of MBP-HRPT2 from Raji cells and Jurkat cells of EXAMPLE 2. 50 µg total protein prepared from MBP- HRPT2 transfected or non transfected cells was separated on an 12% SDS PAGE and transfered onto nitrocellulose. Membrane was probed with anti-MBP antisera (NEB).

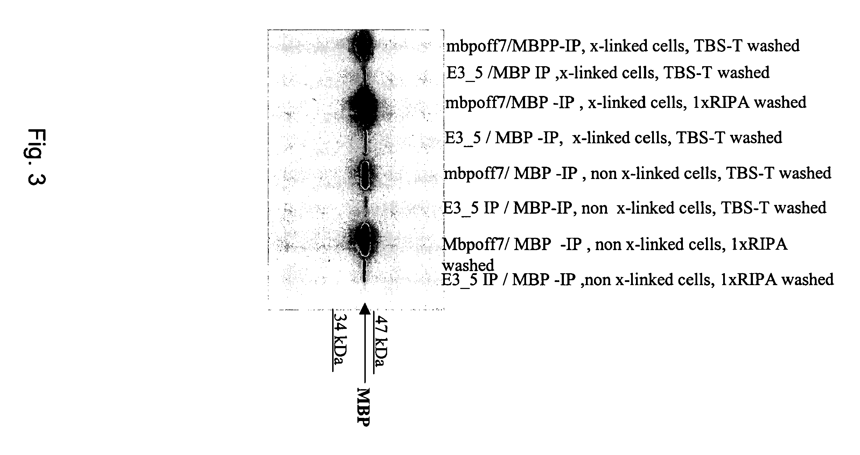

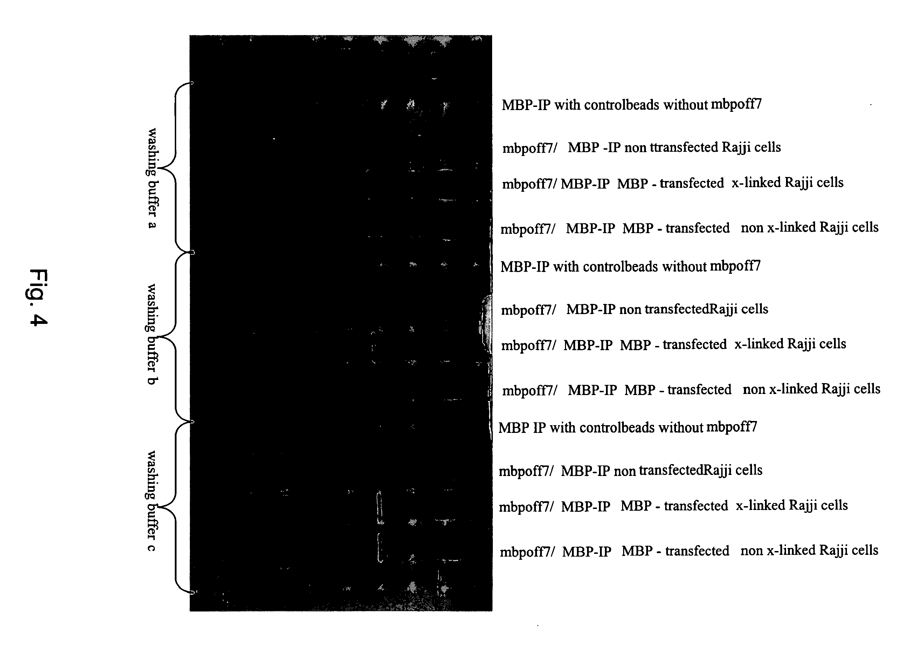

- **Fig.3 and 4**: show a Western blot analysis of a MBP IP using mbpoff7 or the unspecific DARP E3_5. Whole cell extracts (50 µg total protein) isolated from Raji cells overexpressing MBP were immunoprecipitated with mbpoff7 or E3_5 as indicated. To monitor MBP anti-MBP antisera was used in the western blot analysis (Fig. 3) Alternatively 20 µl of the samples were analyzed by SDS PAGE and silver staining (Fig. 4).

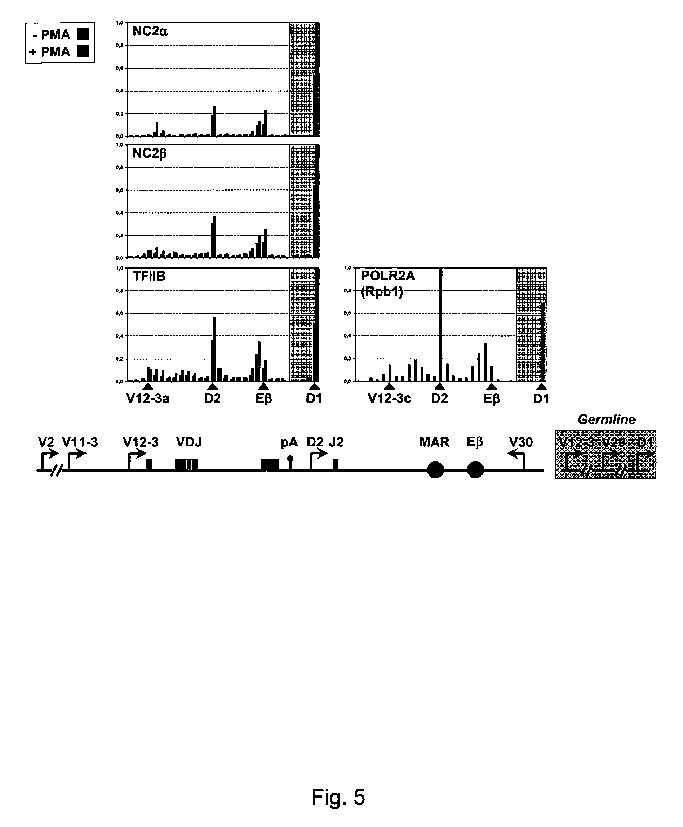

- **Fig. 5**: shows transcription complexes at TCRβ. Specificity of NC2α Mab in ChIP: ChIP experiments of NC2alpha I comparison to NC2β, TFIIB and Rpbl and distribution of the complexes over the TCRβ promoter. 20 ng DNA template, resulting from the corresponding ChIP experiments, was analyzed by real-time-PCR using primer pairs representing different TCRβ promoter regions as indicated in the figure (- PMA and + PMA: first and second column of each condition, respectively).

### EXAMPLES

### EXAMPLE 1

### Heterologous expression and purification of the Designed Ankyrin Repeat Protein mbpoff7 (DARP mbpoff7)

The DARP mbpoff7 was expressed and purified as described in Binz et al., 2003, J. Mol. Biol. 332, 489-503. SDS PAGE of the purification of the DARP mbpoff7. Critical steps during the purification of the recombinant protein mbpoff7 (as indicated) were analyzed on a 12 % SDS gel following a coomassie blue staining.

### EXAMPLE 2

### Expression of MBP and MBP-tagged protein in human cells

The MBP sequence was cloned as an N-terminal tag in the expression vector pREP4. For transfection of the construct of about 5 x 10⁶ Raji cells were pelleted and washed once with PBS. Cells were resuspended in 400 µl growth medium (RPMI) without serum per sample. The cell suspension was then distributed in a precooled electroporation cuvette (Gene Pulser Cuvette, BioRad Cat. N. 165-2088) where it was mixed together with a total amount of 15µg DNA and left on ice for about 10-20 minutes.

For the electroporation the cuvette was connected to a Gene Pulser electroporator (BioRad) and the conditions set to a capacitance of 1000 µF and a voltage of 230 V.

After the electric shock, cells were resuspended in 5 ml medium per sample, transferred in 25 cm² flasks and incubated at 37°C and 5% CO₂. 48 hours later, cells were harvested and pellets were resuspended in 200 µl 1 x GENNT buffer (5% glucose, 5 mM EDTA, 0.2% Nonidet P40, 150mM NaCl, 50 mM Tris-Hcl pH 8.0, containing protease inhibitors). Samples were incubated for 15 minutes on ice, vortexed and centrifuged for 10 minutes at 13200 r.p.m. at 4°C. The supernatant was transferred in fresh tubes, protein concentration was estimated by Bradford and 50 µg total protein were subjected to western blot analysis.

### EXAMPLE 3

### Immunoprecipitation of MBP out of Raji cell extracts with or without previous crosslinking of the cells

0.5 x 10⁸ stable Raji-MBP cells were harvested for 5 minutes at 1200 r.p.m. and washed once in growth media (RPMI) without serum. The pellet was resuspended in 2.5 ml RPMI media, formaldehyde (37%) was added to a concentration of 1% and cells were shaken on a roller for 9 minutes at room temperature. The reaction was stopped by adding 125 mM glycine and incubation on ice for 10 minutes. Pellets were washed three times in ice cold PBS, resuspended in 5 ml 1 x RIPA buffer (1% NP40, 0.5% deochycolate, 0,1% SDS in PBS, containing protease inhibitors), incubated 10 minutes on ice and sonicated three times for 15 seconds at 20% output setting on an ice/ethanol water bath using a Branson 250 sonifier. The suspension was divided in 2 ml tubes and centrifuged for 15 minutes at 13200 r.p.m at 4°C. Supernatants were transferred in fresh tubes and protein concentration was estimated by Bradford assay.

For extracts from non crosslinked cells the same amount of cells were instead of crosslinking kept on ice and proceeded than the same way as the crosslinked cells.

For preparation of the mbpoff7/E3_5 beads 40 µl Affigel 15 (BioRad) beads were used per reaction. Beads were pretreated following the instructions of the manufactor and incubated over night with 10 µg mbpoff7 per reaction in a total volume of 500 µl TBS₅₀₀ (50 mM Tris-HCl, pH 8.0, 500 mM NaCl) on a roller at 4°C. Beads were blocked by adding 0.1 ml 1 M glycinetehylester per ml gel and incubation for 1 h at 4°C on a roller. Beads were washed with 5 ml ice cold H₂O and equilibrated with 0.5 x RIPA buffer. The equilibrated beads were incubated with 125 µg protein extract and incubated over night at 4°C on a roller. Beads were washed 3 times with 1 ml of washing buffer (TBS-T; 1 x RIPA buffer; 250mM LiCl, 1% NP40, 1% deoxycholate (a); 500mM LiCl, 1% NP40, 1% deoxycholate (b) or 500mM LiCl, 1% NP40, 1% deoxycholate, 0,1% SDS (c) as indicated. Finally, beads were resuspended in 30 µl 2 x protein loading dye, incubated for 5 minutes at 95°C and 15µl supernatant were subjected to western blot analysis.

### EXAMPLE 4

### NC2 ChIP experiments

NC2 ChIP experiments were carried out according to the following protocol adapted from Odom et al., 2004, Science 303, 1378-1381.
(See also http://web.wi.mit.edu/young/pancregulators/).

### 1. Induction of cells

- Induce cells with 4-OHT (final concentration 1 µM).

### 2. Cell Cross-Linking

- Use 1x10⁸ to 1x10⁹ cells. Split cells the day before harvest. Harvest cells at appropriate cell density (e.g. 5x10⁵ cells/ml for Jurkat).
- Pool cells, take small aliquot (≤ 100 µl) into Eppendorf cup and resuspend w/ P200 to obtain single cells for counting. Determine cell density and total cell number in Neubauer counting chamber (cell number in 16 small squares x 10⁴ = cell number/ml).
- Spin cells down in 250 ml Coming tubes, 1200 rpm, 5 min, RT.
- Remove supernatent, resuspend cell pellets in ~ 40 ml pre-warmed medium (w/ 10% serum) and pool into 50 ml Falcon tube.
- Spin down, 1200 rpm, 5 min, RT.
- Remove supernatent, resuspend pellet in 43.78 ml medium w/o serum (RT)
- Add 37% formaldehyde 1.22 ml 37% CH₂O -> 1% final conc.(Roth, p.a., # 4979.1), total volume 45.00 ml.
- Incubate on roller table for 9 min at RT, constant agitation.
- Add 2 M glycine 3.00 ml 2 M glycine -> 125 mM (filter sterilized) final conc, total volume 48.00 ml.
- Mix quickly, put on ice for 10 min (suspension will turn yellow upon addition of glycine).
- Work on ice from here on.
- Spin down, 1200 rpm, 5 min, 4°C.
- Remove supernatent, wash pellet 3 x 50 ml ice-cold PBS.
- Last wash: transfer pellet to 15 ml Falcon tube, spin, remove PBS as much as possible, freeze pellet on dry ice and store at -80°C. Alternatively, continue after last wash directly with step 0.

### 3. Cell Lysis and Sonication

- Add Complete (Roche, # 11697498001) protease inhibitor mix to all lysis buffers before use.
- **25 x Complete**: Grind 1 tablet in between 2 sheets of balance paper, dissolve fine powder in 2 ml of H₂O, aliquot and store (labeled 25xC) at -20°C.
- **Lysis Buffer 1 (LB1)** (final conc.): 50 mM Hepes-KOH (pH 7.5), 140 mM NaCl, 1 mM EDTA (pH 8.0), 10 % glycerol, 0.5 % NP-40, 0.25 % 10% Triton X-100
- **Lysis Buffer 2 (LB2)** (final conc.): 200 mM NaCl, 1 mM EDTA, 0.5 mM EGTA, 10 mM Tris-HCl (pH 8)
- **Lysis Buffer 3 (LB3)** (final conc.): 140 mM NaCl, 1 mM EDTA, 0.5 mM EGTA, 10 mM Tris-HCl (pH 8), 0.5 % N-lauroyl sarcosine before use supplement with (final conc.):
   BEFORE sonication: 0.1 % Na-deoxycholate (freshly prepared) and 1 x complete
   AFTER sonication: 0.5 % Triton X-100 and 10% glycerol
- When using frozen pellets: thaw on ice; when using fresh pellets: add directly 10 ml of Lysis Buffer 1 (LB 1) per pellet. Mark pellet volume on tube to check for efficient nuclei prep in step 0.
- Rock at 4°C for 10 min. Then spin at 4000 rpm, 10 min, at 4°C (e.g. in Heraeus Multifuge). Pellet should be smaller (check marks on tube) and look brighter. If this does not happen, repeat step 0. Eventually check nuclei in microscope.
- Resuspend each tube of pellets in 10 ml Lysis Buffer 2 (LB2). Rock gently at RT for 10 min. Then spin at 4000 rpm, 10 min, at 4°C.
- Resuspend pellet in 3 ml buffer 1x Lysis Buffer 3 (1x LB3) without Triton X-100 and glycerol. Sonicate the suspension in 15 ml conical tube with a microtip attached to Branson 250 sonifier on ice/ethanol water bath:
- Sonifier settings:
   Slow mode: 40% output setting, time 30 sec, Pulse On 0,9 sec, Pulse Off 0,1 sec Sonicate 13 cycles, i.e. 6 min 30 sec in total, allowing the suspension to cool on ice for ≥ 1 min between pulses.
- After sonication, add 1/20 volume of 10% Triton X-100 to 0,5% final concentration. Transfer to 1.5 ml tubes and spin out debris at 4°C, 15 min, 13 k rpm in cooled tabletop centrifuge.
- Transfer supernatant (= soluble chromatin) to fresh 15 ml conical tube. Determine "dsDNA" conc. of sample (≥ 1:100 dilution for measurement). Dilute chromatin solution to 1 mg/ml (if possible to 2 mg/ml) "dsDNA" Conc. with LB3 and add glycerol to 10% final conc. Transfer 1 ml aliquots to 1,5 ml tubes, set 10 µl aliquot aside for analysis, and snap-freeze 1 ml aliquots in liquid nitrogen.
- Store at -80°C.
- Analyze 10 µl sample from 2.6 for chromatin size: add 30 µl (3 volumes) of ChIP EB (see 3.x) to 10 µl sample. Add 2 µl of proteinase K (PCR grade; Roche) and incubate ≥ 2 hours at 65°C to revert crosslinks and digest proteins. Purify DNA w/Qiaquick PCR purification kit (column format). Load 1 to 2 µg purified DNA onto 1% agarose gel and check for size distribution of fragments.
- If necessary (too large bulk size of fragments), a second sonication might be performed. Settings for this second sonication have to be determined empirically. Be aware that samples now contain 0.5% Triton X-100 from step 2.5, making the chromatin solution more prone to foaming during the second sonication.

### 4. Immunoprecipitation

- Mix Prot.A/G-Sepharose (FF, GE Healthcare) in a ration of 1:2 due to different binding capacities.
- Wash beads 2-3x with icecold 1xPBS in lubricated tubes (centrifuge ≤ 3k rpm at 4°C for 1').
- Block beads by incubation with 1xPBS / 0,5% for 30' at 4°C rocking.
- Thaw sufficient aliquots of X-linked chromatin samples. Per ChIP calculate to use 100 µl of 2 mg/ml or 200 µl of 1mg/ml X-linked chromatin extract. Thus, an aliquot of 1 ml is sufficient for 5 ChIPs (if 1 mg/ml conc.) or 10 ChIPs (if 2 mg/ml conc.).
- Pre-clear chromatin extract by incubating with blocked beads: use 10 µl (dbv) of appropriate beads per 100 µl of chromatin extract. Thus, a total of 100 µl (dbv) of blocked beads is used per 1 ml aliquot of chromatin extract. Add 200 µl of 50% blocked beads slurry (in 1x LB3) per 1 ml aliquot. Incubate with constant agitation at 4°C for 30-60 min (lubricated tubes).
- In parallel, pre-coat blocked beads with appropriate antibodies. Per ChIP typically 2-10 µg antibody is used; if working with hybridoma tissue culture sups, use 100-200 µl of sup instead. Use 15-20 µl (dbv) of appropriate beads per ChIP.
- Assemble precoating mix (in lubricated tubes): 40 µl 50% blocked beads PBS slurry(= 20 µl dbv blocked beads), x µl antibody (= 1 µg), add 400 µl w/ cold PBS
- Incubate with constant agitation at 4°C for 30-60 min.
- Spin down 1 min at 4°C, 3000 rpm, and remove sup. Wash pellet 1x w/ 1 ml cold 1xPBS. Spin down, remove sup. Resuspended pellet in 1 volume 1x LB3 (0.5% Triton X-100, 1x Complete, but w/o glycerol).
- Assemble ChIP sample (in lubricated tube): 40 µl antibody-coated beads (50% slurry in 1x LB3), 100 µl (or 200 µl) of pre-cleared chromatin extract (from 0)
- Incubate ChIP samples for ≥ 2 hours (typically o/n) with constant agitation at 4°C.

### 5. Washing, eluting, and reverse cross-linking

- Spin beads, 3000 rpm, 30 sec, 4°C
- Wash beads 6x with 1ml 1x RIPA-Buffer
- **Wash buffer (2x RIPA buffer)** (final conc., if 1x): 50 mM Hepes (pH 7.6), 1mM EDTA, 1 % NP-40 (IPGEL), 0.5 M LiCl
   Add to freshly prepared 1x RIPA-buffer: 0,1 % Na-deoxycholate (freshly prepared), 1x Complete
- Wash beads 1x with 1 ml TE-buffer + 50mM NaCl
- Add 100 µl prewarmed (65°C) Elution buffer and rock samples for 10 min at 1400 rpm and 65°C in thermomixer.
- **Elution buffer:** 50mM Tris pH 8.0, 10 mM EDTA, 1% SDS
- Spin down beads, 13k rpm, 1 min, RT; transfer all of the 100 µl sup in 1,5 ml non-lubricated tubes.
- Fill Inputs ad 100 µl with H₂O and add 3x volumes of Elution buffer.
- Reverse x-link O/N 65°C.

### 6. RNase A, Proteinase K

- Add 1x volume of TE-buffer and then add RnaseA (1mg/ml) so final is ~0.05 µg/µL: 5µl per ChIP and 10µl per Input. Incubate for 1-2 h at 37°C.
- Add proteinase K so final is 0.2 µg/µL (~2.5 µL/250 µL rxn): 4 µl per ChIP and 8 µl per Input.
- Incubate for 2 h at 56°C.
- Store at -20°C. Alternatively, continue directly with step 0.

### 7. DNA-purification

- Add once 1x vol of PCI-mix (25:24:1, Fa. Roth) and vortex 20-30 sec.
- Spin samples, 13k rpm, RT, 5 min and transfer aqeuos (upper) phase in fresh 1,5 ml tube.
- Prepare mastermix for EtOH-precipitation: 1/25x vol 5 M NaCl, 1,5 µl Glykogen, 2x vol 100% EtOH, icecold, add mastermix to samples, mix and store samples at -80°C (≥ 30 min) or -20°C (≥ 2 h).
- Spin samples, 13k rpm, RT, 5 min and discard supernatant. Wash pellet with 500 µl ice-cold 70% EtOH and spin again 13k rpm, RT, 5 min.
- Dry pellet, then resuspend in 50 µl 10 mM Tris/HCl. Rock samples for 15 min at RT in the thermomixer.
- Determine "dsDNA" conc. of sample (≥ 1:100 dilution for measurement). Load 0.5 to 2 µg purified DNA onto 1% agarose gel and check for size distribution of fragments.

### 8. ChIP-analysis

- Use ~20 ng as template in the PCR-rxn.

## Claims

1. An affinity marker comprising
- a first tag comprising a first epitop for an IgG antibody, wherein the first epitope is derived from a cellular protein; and
- a second tag comprising a second epitop for an artificial antibody.

2. The affinity marker of claim 1, wherein the first epitope is derived from a cellular protein of a eukaryotic cell, preferably a vertebrate cell, more preferably a mammalian cell, still more preferably a human cell.

3. The affinity marker of claim 1 or 2, wherein the cellular protein is not present in prokaryotic cells.

4. The affinity marker of any of claims 1 to 3, wherein the cellular protein is a protein capable of binding DNA, optionally a complex of DNA and one or more proteins, more preferably the TATA box binding protein (TBP) -TATA Element Complex.

5. The affinity marker of any of claims 1 to 4, wherein the cellular protein is derived from negative co-factor 2 (NC2), preferably NC2α.

6. The affinity marker of any of claims 1 to 5, wherein the first tag comprises or consists of the sequence Met Ser Pro Pro Thr Pro Phe Leu Pro Phe Ala Ser Thr Leu Pro Leu Pro Pro Ala Pro Pro (SEQ ID NO:1).

7. The affinity marker of claim 6, wherein the first tag differs from the sequence of SEQ ID NO:1 by at most 5, preferably at most 4, more preferably at most 3, still more preferably at most 2, most preferably at most 1 amino acid addition, deletion and/or substitution.

8. The affinity marker of claim 7, wherein the amino acid substitution(s) is/are (a) conservative amino acid substitution(s).

9. The affinity marker of any of claims 1 to 8, wherein the second epitope is derived from a protein which is not present in a human cell, preferably a mammalian cell, more preferably a vertebrate cell, still more preferably an animal cell.

10. The affinity marker of any of claims 1 to 9, wherein the second epitope is derived from a protein of a fungal cell, preferably a yeast cell, more preferably from *Saccharomyces cerevisiae.*

11. The affinity marker of any of claims 9 or 10, wherein the protein is a transcription factor, preferably a transcription factor responsible for the activation of one or more genes required for amino acid or for purine biosynthesis.

12. The affinity marker of any of claims 1 to 11, wherein the second epitope is derived from GCN4.

13. The affinity marker of any of claims 1 to 12, wherein the second tag comprises or consists of the sequence Arg Met Lys Gln Leu Glu Pro Lys Val Glu Glu Leu Leu Pro Lys Asn Tyr His Leu Glu Asn Glu Val Ala Arg Leu Lys Lys Leu Val Gly Glu Arg (SEQ ID NO:2).

14. The affinity marker of claims 1 to 8, wherein the second epitope is an epitope for an ankyrin repeat protein.

15. The affinity marker of claim 14, wherein the epitope for an ankyrin target protein comprises or consists of the sequence of the Maltose-binding protein (MBP) tag, particularly the sequence encoded by the sequence

AGCTTGCGGCCATGAAAACTGAAGAAGGTAAACTGGTAATCTGGATTAA
CGGCGATAAAGGCTATAACGGTCTCGCTGAAGTCGGTAAGAAATTCGAG

AAAGATACCGGAATTAAAGTCACCGTTGAGCATCCGGATAAACTGGAAG
AGAAATTCCCACAGGTTGCGGCAACTGGCGATGGCCCTGACATTATCTTC
TGGGCACACGACCGCTTTGGTGGCTACGCTCAATCTGGCCTGTTGGCTGA
AATCACCCCGGACAAAGCGTTCCAGGACAAGCTGTATCCGTTTACCTGGG
ATGCCGTACGTTACAACGGCAAGCTGATTGCTTACCCGATCGCTGTTGAA
GCGTTATCGCTGATTTATAACAAAGATCTGCTGCCGAACCCGCCAAAAAC
CTGGGAAGAGATCCCGGCGCTGGATAAAGAACTGAAAGCGAAAGGTAA
GAGCGCGCTGATGTTCAACCTGCAAGAACCGTACTTCACCTGGCCGCTGA
TTGCTGCTGACGGGGGTTATGCGTTCAAGTATGAAAACGGCAAGTACGA
CATTAAAGACGTGGGCGTGGATAACGCTGGCGCGAAAGCGGGTCTGACC
TTCCTGGTTGACCTGATTAAAAACAAACACATGAATGCAGACACCGATTA
CTCCATCGCAGAAGCTGCCTTTAATAAAGGCGAAACAGCGATGACCATC
AACGGCCCGTGGGCATGGTCCAACATCGACACCAGCAAAGTGAATTATG
GTGTAACGGTACTGCCGACCTTCAAGGGTCAACCATCCAAACCGTTCGTT
GGCGTGCTGAGCGCAGGTATTAACGCCGCCAGTCCGAACAAAGAGCTGG
CAAAAGAGTTCCTCGAAAACTATCTGCTGACTGATGAAGGTCTGGAAGC
GGTTAATAAAGACAAACCGCTGGGTGCCGTAGCGCTGAAGTCTTACGAG
GAAGAGTTGGCGAAAGATCCACGTATTGCCGCCACTATGGAAAACGCCC
AGAAAGGTGAAATCATGCCGAACATCCCGCAGATGTCCGCTTTCTGGTAT
GCCGTGCGTACTGCGGTGATCAACGCCGCCAGCGGTCGTCAGACTGTCGA
TGAAGCCCTGAAAGACGCGCAGACTGGATC
(SEQ ID NO:3).

16. The affinity marker of claim 13 or 15, wherein the second tag differs from the sequence of SEQ ID NO:2 or the sequence encoded by the sequence of SEQ ID NO:3 by at most 10, preferably at most 5, more preferably at most 3, still more preferably at most 2, most preferably at most 1 amino acid addition, deletion and/or substitution.

17. The affinity marker of claim 16, wherein the amino acid substitution(s) is/are (a) conservative amino acid substitution(s).

18. The affinity marker of any of claims 1 to 17, wherein the first tag and the second tag are connected by a linker.

19. The affinity marker of claim 18, wherein the linker consists of 1, 2, 3, 4, 5, 6, 7, 8, 9 or 10 amino acids, preferably 1, 2, 3, 4 or 5 amino acids, more preferably 1, 2 or 3 amino acids.

20. The affinity marker of claim 18 or 19, wherein the linker consists of Gly and Ser.

21. The affinity marker of any of the claims 18 to 20, where the linker comprises or consists of the amino acid sequence Gly Ser Gly.

22. The affinity marker of any of claims 1 to 7, 9 to 13, 16 and 18 to 21 comprising or consisting of the sequence Met Ser Pro Pro Thr Pro Phe Leu Pro Phe Ala Ser Thr Leu Pro Leu Pro Pro Ala Pro Pro Gly Ser Gly Arg Met Lys Gln Leu Glu Pro Lys Val Glu Glu Leu Leu Pro Lys Asn Tyr His Leu Glu Asn Glu Val Ala Arg Leu Lys Lys Leu Val Gly Glu Arg (SEQ ID NO:4).

23. The affinity marker of any of claims 1 to 22 additionally comprising at least one cleavage site, preferably a protease cleavage site, more preferably a TEV or PreScisson cleavage site.

24. A protein comprising the affinity marker according to any of claims 1 to 23.

25. A nucleic acid coding for the affinity marker according to any of claims 1 to 23 or the protein according to claim 24.

26. A vector comprising the nucleic acid according to claim 25.

27. A cell comprising the nucleic acid according to claim 25 and/or the vector according to claim 26.

28. A method of determining or localizing a nucleic acid sequence comprising
a) incubating the protein according to claim 24 with a nucleic acid under conditions allowing the formation of a protein nucleic acid complex;
b) optionally producing fragments of the nucleic acid of the complex of step a);
c) purifying the complex of step a) or b) by means of affinity purification using an artificial antibody against the second epitope; and
d) determining and/or localizing the nucleic acid sequence bound to the protein,
wherein the first epitope is used for pre-purification of the protein nucleic acid complex of step a) or b) prior to step c) and/or is used for standardization.

29. A method of purifying a protein comprising
d) providing the protein according to claim 24;
e) purifying the protein of step a) by means of affinity purification using an IgG antibody; and
f) purifying the purified protein from step b) by means of affinity purification using an artificial antibody.

30. A method of purifying a protein comprising
d) providing the protein according to claim 24;
e) purifying the protein of step a) by means of affinity purification using an artificial antibody; and
f) purifying the purified protein from step b) by means of affinity purification using an IgG antibody.

31. The method of claim 28 to 30, wherein the artificial antibody is a single chain antibody or an ankyrin repeat protein.

32. The method of claim 31, wherein the single chain antibody is the scFv antibody 52SR4.

33. The method of any claims 28 to 32, wherein the IgG antibody binds said epitope with an affinity at most 100 x 10⁻⁹ mol/l, preferably at most 50 x 10⁻⁹ mol/l, more preferably 10 x 10⁻⁹ mol/l, still more preferably 5 x 10⁻⁹ mol/l, even more preferably 3 x 10⁻⁹ mol/l, most preferably 1 x 10⁻⁹ mol/l.

34. The method of any claims 28 to 33, wherein the artificial antibody binds said epitope with an affinity at most 100 x 10⁻¹² mol/l, preferably at most 50 x 10⁻¹² mol/l, more preferably 10 x 10⁻¹² mol/l, still more preferably 5 x 10⁻¹² mol/l, even more preferably 3 x 10⁻¹² mol/l, most preferably 1 x 10⁻¹² mol/l.

35. The method of any of claims 28 to 34, wherein the protein to be purified has been produced by a eukaryotic cell, preferably a vertebrate cell, more preferably a mammalian cell, still more preferably a human cell.

36. The method of any claims 28 to 35, wherein at least one of the tags is cleaved off after step b), c) and/or step d).

37. The method of any of claims 28 to 36, wherein the IgG antibody and/or the artificial antibody is/are attached to a microarray or chip.

38. The method of any of claims 28 to 37, wherein the first epitope is used for standardization and/or as recovery marker.

39. Use of the affinity marker according to claims 1 to 23 for the purification of a protein and/or for determining or localizing a protein or nucleic acid sequence.

40. A kit comprising
- the nucleic acid according to claim 25 and/or the vector according to claim 26; and
- optionally an IgG antibody specific for the first epitope; and
- optionally an artificial antibody specific for the second epitope.
